# EUROPEAN PATENT SPECIFICATION

(11) **EP 1 888 089 B1**
(45) Date of publication and mention of the grant of the patent: **08.06.2011**
(21) Application number: 06751864.7
(22) Date of filing: 28.04.2006
(51) Int. Cl.: C12Q 1/68, A61K 36/00

(54) **EX VIVO GENE EXPRESSION IN WHOLE BLOOD AS A MODEL OF ASSESSMENT OF INDIVIDUAL VARIATION TO DIETARY SUPPLEMENTS**
EX-VIVO-GENEXPRESSION IN VOLLBLUT ALS MODELL FÜR DIE BEURTEILUNG VON INDIVIDUELLER VARIATION GEGENÜBER NAHRUNGSZUSÄTZEN
EXPRESSION DE GENES EX VIVO DANS LE SANG ENTIER COMME MODELE D'EVALUATION DE LA VARIATION DE REACTION INDIVIDUELLE A DES COMPLEMENTS DIETETIQUES

(30) Priority: 28.04.2005 US 675580 P
(43) Date of publication of application: 20.02.2008
(73) Proprietor: HITACHI CHEMICAL RESEARCH CENTER, INC., Irvine, CA 92617 (US); Hitachi Chemical Co., Ltd., Tokyo 163-0449 (JP)
(72) Inventor: MITSUHASHI, Masato, Irvine, CA 92614 (US)
(74) Representative: Bannerman, David Gardner
(86) International application number: PCT/US2006/016376
(87) International publication number: WO 2006/116721

(56) References cited:
- EP-A- 1 243 274
- WO-A-00/76492
- WO-A-03/059333
- WO-A-2005/115115
- US-A- 5 683 698
- US-A1- 2002 006 613
- US-A1- 2002 106 684
- AMES B.N. AND WAKIMOTO P.: 'Are vitamin and mineral deficiencies a major cancer risk?' NATURE vol. 2, 2002, pages 694 - 704, XP003002464

## Description

### BACKGROUND OF THE INVENTION

### Field of the Invention

The present invention relates to a method for tailoring the administration of dietary components such as supplements. In the method, whole blood of a mammal is exposed to a dietary component. The level of a marker mRNA linked to a disease state is measured in leukocytes after exposure to the dietary component, and in some cases after further stimulation of the exposed blood cells. By comparing the mRNA level after exposure with the value found in unexposed blood cells, it is possible to determine what the effect of the dietary component will be in the mammal. By screening blood of the mammal against a number of possible dietary components, it is possible to develop an optimized set of dietary components tailored to the specific mammal to treat or prevent a disease state.

### Description of the Related Art

Dietary components (supplements), such as vitamins, polyphenols, turmeric, etc. are known to induce various biological activities in cultured cells and animals, and some of these activities have confirmed by subsequent clinical studies. Some of these biological activities would be expected to have an effect on patient outcomes in various disease states. For example, those dietary components that increased activity of components of the immune system might be expected to have an effect against some cancers, while dietary components that decreased activity of certain immune system components might be efficacious in cases of autoimmune disease. However, it is difficult to employ clinical study results to design an individualized combination of dietary components for a particular individual. If both responders and non-responders exist in a study population, and the non-responder population is substantially larger than the responder population, double-blind clinical trials are no longer capable of identifying those dietary components that can be expected to have efficacy. Furthermore, genotyping or single nucleotide polymorphism analysis is only useful in dietary optimization once target genes and hot spots have been characterized. The value of so-called tailored, individualized, or personalized medicine or nutrition is realized both among the scientific community and the general public (see Jain, KK, 'Personalized medicine," Curr Opin Mol Ther 2002; 4: 548-58). However, the applicable technology is still limited.

Various reports have already shown the blood levels of dietary supplements. It would be desirable to compare the known or suspected effects of various dietary supplements at the known standard blood levels to the actual individual results of a mammal under consideration for dietary therapy. This would allow the efficacy of each supplement in leukocytes of the mammal from which the whole blood was drawn to be assessed, with a view to designing a diet or set of dietary supplements for the mammal. These effects in leukocytes will be applicable to inflammation, cancer immunity, autoimmune diseases, and the like. However, there has not as yet been an effective method for accomplishing this, particularly in an ex vivo context.

EP 1 243 274 relates to a method of preventing or treating diseases by administering a fermented soy extract FSE to the patient. Soy extract is particularly useful in preventing or treating cancer, infections, asthma and inflammation. The fermented soy extract is made form the fermentation of an aqueous soy extract with at least one lactic acid bacteria.

US 5,683,698 relates to a formulation and to the use of the formulation in the reduction and alleviation of symptoms associated with arthritis. The formulation comprises a mixture of alpinia, smilax, tinospora, tribulus, withania and zingiber each in a physiologically acceptable amount.

US 2002/006613 relates to the identification and markers that can be used to determine whether cancer cells are sensitive or resistant to a particular therapeutic agent. A method for identifying therapeutic targets is also described.

### SUMMARY OF THE INVENTION

The present invention discloses a method for tailoring dietary components such as supplements to individual mammals based on the levels of marker mRNA measured in leukocytes after exposure of whole blood of the mammal to candidate dietary components.

In an embodiment of the present invention, a method is provided of assessing the potential effectiveness of a dietary component in an individual mammal against cancer or an autoimmune disorder, comprising:
exposing whole blood of the mammal to the dietary component and a stimulating agent selected from phytohemagglutinin and heat-aggregated IgG *in vitro;*
measuring the amount of an mRNA in leukocytes of the exposed whole blood, and unexposed whole blood, said mRNA being associated with the cancer or an autoimmune disorder; and
identifying the potential effectiveness of the dietary component in the mammal based on the results of the measurement, wherein a change in the amount of the mRNA
determined by comparing the amount of the mRNA measured in leukocytes of the unexposed whole blood wich the amount of the mRNA measured in leukocytes of the exposed whole blood correlates with the potential effectiveness of the dietary component,
wherein the mRNA is selected from mRNAs encoding interleukin-2, interleukin-4, tumor necrosis factor alpha, IgG Fc receptor, p21, Fas ligand, tumor necrosis factor superfamily member 3, and tumor necrosis factor superfamily member 15.

In a further aspect, the method additionally comprises: after exposure, exposing the whole blood to a stimulating agent; and assessing the potential effectiveness of a dietary component includes comparing results of the measurement obtained from unexposed whole blood with results of the measurement obtained after exposure to the dietary component and stimulating agent.

The stimulating agent is selected from the group consisting of phytohemagglutinin, radiation, and heat-aggregated IgG.

In a further aspect, the unexposed whole blood is exposed to a control vehicle before the amount of mRNA is measured.

In a further aspect, the control vehicle is phosphate-buffered saline or dimethyl sulfoxide.

In a further aspect, exposing whole blood includes addition of heparin.

In a further aspect, the whole blood is stimulated for 5 hours or less.

In a further aspect, the whole blood is stimulated for 30 minutes to 4 hours.

The mRNA is selected from the group consisting of mRNAs encoding interleukin-2, interleukin-4, tumor necrosis factor alpha, IgG Fc receptor, p21, Fas ligand, tumor necrosis factor superfamily member 3, and tumor necrosis factor superfamily member 15.

In a further aspect, the dietary component is selected from the group consisting of vitamin A, vitamin C, vitamin D, vitamin E, epigallocatechin gallate, g-linoleic acids, genistein, curcumin, quercetin, aged garlic, Agaricus, propolis, meshimakobu, noni extract, allcoxyglycerol, and fucoidan.

Also described is a method of measuring the potential anti-cancer effectiveness in a mammal of a dietary component selected from the group consisting of vitamin A, vitamin C, vitamin D, vitamin E, epigallocatechin gallate, g-linoleic acids, genistein, curcumin, quercetin, aged garlic, agaricus, propolis, meshimakobu, noni extract, allcoxyglycerol, and fucoidan, comprising: exposing whole blood of the mammal to the dietary component for 4 hours or less; measuring the amount of mRNA encoding an IgG Fc receptor in blood cells of the exposed whole blood and unexposed whole blood; comparing results of the measurement obtained in blood cells of the exposed whole blood and the unexposed whole blood; and identifying potential anti-cancer effectiveness of the dietary component based on the results of the comparison, wherein a change in the amount of the mRNA correlates with the effectiveness of the dietary component.

Described is a method of measuring the potential anti-cancer effectiveness in a mammal of a dietary component selected from the group consisting of vitamin A, vitamin C, vitamin D, vitamin E, epigallocatechin gallate, g-linoleic acids, genistein, curcumin, quercetin, aged garlic, agaricus, propolis, meshimakobu, noni extract, alkoxyglycerol, and fucoidan, comprising: exposing whole blood of the mammal to the dietary component for 4 hours or less; stimulating the exposed whole blood and unexposed whole blood of the mammal with radiation; after the stimulus, measuring the amount of mRNA encoding the p21 or PUMA gene product in blood cells of the exposed whole blood and the unexposed whole blood; comparing results of the measurement obtained in blood cells of the exposed whole blood and the unexposed whole blood; and identifying potential anti-cancer effectiveness of the dietary component based on the results of the comparison, wherein a change in the amount of the mRNA correlates with the effectiveness of the dietary component.

Described is a method of measuring the potential anti-cancer or anti-autoimmune disorder effectiveness in a mammal of a dietary component selected from the group consisting of vitamin A, vitamin C, vitamin D, vitamin E, epigallocatechin gallate, g-linoleic acids, genistein, curcumin, quercetin, aged garlic, agaricus, propolis, meshimakobu, noni extract, alkoxyglycerol, and fucoidan, comprising: exposing whole blood of the mammal to the dietary component for 4 hours or less; stimulating the exposed whole blood and unexposed whole blood of the mammal with phytohemagglutinin; after said stimulus, measuring the amount of mKNA encoding a protein selected from the group consisting of interleukin-2, interleukin-4, tumor necrosis factor alpha, and Fas ligand in blood cells of the exposed whole blood and the unexposed whole blood; comparing results of the measurement obtained in blood cells of the exposed whole blood and the unexposed whole blood; and identifying potential anti-cancer or anti-autoimmune disorder effectiveness of the dietary component based on the results of the comparison, wherein a change in the amount of the mRNA correlates with the effectiveness of the dietary component.

### BRIEF DESCRIPTION OF THE DRAWINGS

Figure 1 shows the results of a measurement of the levels of IL-2, IL-4, and TNF-α mRNA in leukocytes after phytohemagglutinin stimulation of whole blood.
Figure 2 shows the results of a measurement of the levels of CD32A mRNA in leukocytes after exposure of whole blood to various dietary components.
Figure 3 shows the results of a measurement of the levels of p21 mRNA in leukocytes after exposure of whole blood to various dietary components and radiation stimulation.
Figure 4 shows the results of a measurement of the levels of Fas ligand mRNA in leukocytes after exposure of whole blood to various dietary components and phytohemagglutinin stimulation.
Figures 5-7 show the results of a measurement of the levels of tumor necrosis factor superfamily members 3 and 15 mRNA in leukocytes after exposure of whole blood to various dietary components and heat-aggregated IgG stimulation.

### DETAILED DESCRIPTION OF THE PREFERRED EMBODIMENT

In embodiments of the present invention, the individual variation in response to various dietary components such as dietary supplements was assessed. "Dietary components" refers to any compound or substance that forms part of the diet of a mammal, while "dietary supplements" indicates those beneficial dietary components, such as vitamins and natural extracts, that are used to supplement the diet of mammals. Heparinized human whole blood was incubated with each dietary component ex vivo, and the changes in gene expression induced by exposure to the dietary components was assessed by quantitating the expression of genes linked to conditions such as cancer, autoimmune diseases, and the like in leukocytes exposed to the dietary supplements, as well as in those not so exposed. In some cases, the whole blood was subjected to stimulation with a stimulating agent such as phytohemagglutanin, radiation, or heat-aggregated IgG ("HAG") before quantitating the mRNA levels. Additionally, for some dietary supplements, before quantitiating the mRNA level in unexposed whole blood, the blood was exposed to the vehicle in which the dietary supplement is normally dissolved.

Some dietary components were found to augment or inhibit gene expression; however, substantial individual-to-individual variation was identified, and this variation was statistically significant. For a given individual, the mRNA changes induced by exposure of the whole blood of the individual to a particular dietary component will be correlated with the potential effectiveness of the dietary component in the prophylaxis or treatment of the condition to which the mRNA is linked. Both positive and negative correlation are possible; positive correlation will indicate that the dietary component may be effective against the condition, while negative correlation will indicate that the dietary component will not be effective in that individual and should not be employed. Either an increase or a decrease in the mRNA level may signal the potential effectiveness of the dietary component against a condition, depending on the nature of the condition and the mRNA that is quantitated. For example, an increase in levels of an mRNA linked to activity of the immune system, such as interleukin-2 (a marker for T-cell activation), may be positively correlated with potential effectiveness against cancer, while a decrease in the level of the same mRNA may be positively correlated with effectiveness against autoimmune diseases. The data obtained from the screening of an individual's blood against a number of dietary components could be used to design a diet optimized to treat or prevent a disease linked to the quantitated mRNA, such as cancer.

This method will be useful in industry to discover universally active components contained in crude natural product. In the field of healthcare, the method will open up new possibilities in the design of individualized dietary therapies.

The present method will be explained in greater detail with respect to embodiments of the invention; however, these embodiments should not be interpreted as limiting the present invention.

### Embodiment 1

In order to assess leukocyte function in as close as possible to physiological conditions, an embodiment of the method of the invention employs whole blood, without isolating specific leukocyte populations. As anticoagulant citrate dextrose solution and ethylenediaminetetraacetic acid chelate calcium, a critical component for many biological activities (see Eggesbo et al., "LPS induced release of IL-1 beta, IL-6, IL-8 and TNF-alpha in EDTA or heparin anticoagulated whole blood from persons with high or low levels of serum HDL," Cytokine 1996; 8: 152-60), heparin was used as an anticoagulant in this embodiment. Since mRNA transcription is an upstream event of protein synthesis and subsequent biological activities, mRNA levels are employed in an embodiment of the present invention as an indication of biological activity associated with the protein encoded by the mRNA. An embodiment of the present invention employs a method of quantitating mRNA that allows the identification of changes in gene expression that are as small as 20-40 % (see Mitsuhashi M., "Absolute quantitation of mRNA in human blood leukocytes as a model for phenotypic gene expression-based diagnostics," Clin Chem, 2006).

In this embodiment, phytohemagglutinin-induced gene expression of interleukin-2 (IL-2), interleukin-4 (IL-4), and tumor necrosis factor-α (TNF-a) were quantitated in the following manner. Fifty µL of heparinized whole blood was incubated with various dietary supplements for a period of 30 minutes. The following supplements were employed: vitamins A, C, and D, epigallocatechin gallate (from green tea), genistein (from soy), and curcumin (from the spice turmeric). Vitamin A is known to stimulate the immune system. Vitamin C has been shown to increase the activity of T cells. Vitamin D appears to have protective effects against certain cancers. Epigallocatechin gallate is a powerful antioxidant, appears to reduce the multidrug resistance found in cancer cells, and preferentially induces apoptosis in neoplastic cells. Genistein has been found in some studies to have anticarcinogenic activity; possible mechanisms of action include upregulation of apoptosis, inhibition of angiogenesis, inhibition of DNA topoisomerase II and inhibition of protein tyrosine kinases. Curcumin has proapoptotic effects in neoplastic cells and interferes with the activity of the transcription factor NF-κB, which is often highly overexpressed in such cells.

After incubation with the dietary supplement, 100 µg/mL phytohemagglutinin (PHA) was added and incubation was continued for an additional 2 hours at 37°C. The concentration of each dietary supplement (see Table I, note) was slightly higher than reported blood levels. Then mRNA was purified, cDNA was synthesized, and the levels of IL-2, IL-4, and TNF-α were quantitated by TaqMan real time polymerase chain reaction (PCR) (see Holland, et al., "Detection of specific polymerase chain reaction product by utilizing the 5' to 3' exonuclease activity of Thermus aquaticus DNA polymerase," Proc Natl Acad Sci USA 1991; 88: 7276-80), as described below.

The mRNA and cDNA were prepared from the whole blood that was exposed to the dietary components, as well as the whole blood which remained unexposed. In brief, homemade 96-well filterplates were placed over collection plates, and 150 µl 5 mM Tris, pH 7.4, was applied. Following centrifugation at 120 xg for 1 min at 4°C, 50 µl of blood samples were applied to each well and immediately centrifuged at 120 xg for 2 min at 4°C, followed by washing of each well with 300 µl PBS once with centrifugation at 2000 xg for 5 min at 4°C. Then, 60 µl stock lysis buffer, containing for example 0.5% N-Lauroylsarcosine, 4X SSC, 10 mM Tris HCl, pH 7.4, 1 mM EDTA, 0.1% IGEPAL CA-630, and 1.791 M guanidine thiocyanate, supplemented with 1% 2-mercaptoethanol (Bio Rad, Hercules, CA, USA), 0.5 mg/ml proteinase K (Pierce, Rockford, IL, USA), 0.1 mg/ml salmon sperm DNA (5 Prime Eppendorf/Brinkmann, Westbury, NY, USA), 0.1 mg/ml E. coli tRNA (Sigma), a cocktail of 10 mM each of the specific reverse primers shown in Table 2, and standard RNA34 oligonucleotides, were applied to the filterplates, followed by incubation at 37°C for 10 min. The filterplates were then placed over oligo(dT)-immobilized microplates (GenePlate, RNAture), and centrifuged at 2000 xg for 5 min at 4°C. Following overnight storage at 4°C, the microplates were washed with 100 µl plain lysis buffer 3 times, followed by 150 µl of wash buffer (0.5 M NaCl, 10 mM Tris, pH 7.4, 1 mM EDTA) 3 times at 4°C. The cDNA was directly synthesized in each well by adding 30 µl of buffer containing lx RT-buffer, 1.25 mM each of dNTP, 4 units rRNasin, and 80 units of MMLV reverse transcriptase (Promega) (without primers), and incubation at 37°C for 2 hours. The specific primer-primed cDNA existed in solution, and oligo(dT)-primed cDNA stayed immobilized in the microplate. For TaqMan PCR, the resultant 4 µl of cDNA solution was directly transferred to 384-well PCR plates, to which 5 µl of TaqMan universal master mix (ABI) and 1 µl oligonucleotide cocktail (15 µM each of forward and reverse primer, and 3-6 µM TaqMan probe) were applied, and PCR was conducted in PRISM 7900HT (ABI), with one cycle of 95°C for 10 min followed by 45 cycles of 95°C for 30 sec, 55°C for 30 sec, and 60°C for 1 min. SYBR Green PCR may also be employed; for this, cDNA was diluted 3-4 fold in water, and 4 µl cDNA solution was directly transferred to 384-well PCR plates, to which 5 µl of a master mix (BioRad, Hercules, CA) and 1 µl of oligonucleotide cocktail (15 µM each of forward and reverse primer) were applied, and PCR was conducted in PRISM 7900HT (ABI), with one cycle of 95°C for 10 min followed by 45 cycles of 95°C for 30 sec and 60°C for 1 min. Each gene was amplified in separate wells. The Ct was determined by analytical software (SDS, ABI).

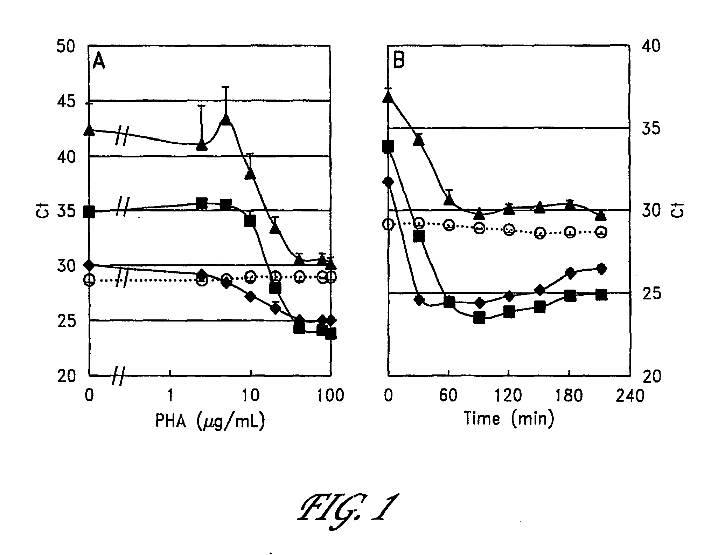

IL-2, IL-4, and TNF-α were chosen as markers of T-cell activation, activation of the IgE cascade (possible allergic reaction), and cytotoxicity, respectively. For accurate statistical analysis (Student's t-test), triplicate aliquots of whole blood were used as the starting materials. Data were expressed as the cycle threshold (Ct), which is the cycle of PCR required to generate certain amounts of PCR products (Figure 1), ΔCt by subtracting Ct values of the un-stimulated samples from PHA-stimulated ones, and ΔΔCT by subtracting ΔCT values of untreated control samples from dietary supplement-treated ones (Table I). Since Ct is a log scale, 1 ΔCt or ΔΔCt means double or one half in quantity, and a negative ΔCt value means an increase in expression.

Figure 1 shows PHA-induced gene expression in whole blood. Fifty µL of heparinized whole blood was incubated with various concentrations (A) or 100 µg/mL (B) of PHA for 2 hours (A) or various lengths of time at 37°C, then the levels of IL-2 (■), IL-4 (▲), and TNFα (◆) were quantitated as described above. Standard artificial RNA (RNA34) (○) spiked into the system was also quantitated. Each data was the mean Ct ± standard deviation from triplicate aliquots of whole blood.

As shown in Figure 1, PHA induced IL-2, IL-4 and TNF-α mRNA expression in a dose dependent manner with an EC₅₀ of approximately 10-20 µg/mL (Figure 1A). The induction of mRNA was rapid and reached a plateau after 30-60 min (Figure 1B). In order to avoid secondary effects on PHA itself, the PHA dose and incubation period were fixed at maximal levels (100 µg/mL and 120 minutes). As shown in Table I, the effect of dietary supplements exhibited substantial individual variation for each mRNA. Since gene expression with 100 µg/mL PHA was over-saturated (Figure 1A), changes less than -0.65 ΔΔCT were remarkable with statistical significance. Interestingly, green tea epigallocatechin gallate (EGCG) enhanced PHA-induced IL-4 expression in all 7 cases, but its action against IL-2 and TNF-α showed individual variations (Table I). For IL-2, 3 out of 7 persons showed decreases, 1 showed an increase, and 3 were unchanged (Table I). Turmeric curcumin (Cur.), significantly decreased IL-2, IL-4, and TNF-α expression in 2 cases, but the other 5 persons showed no changes (Table I). Vitamin A, C, and D all enhanced IL-4 expression, with 1-3 exceptions (Table I). Soybean genistein (labled "Gen" in Table 1) was mainly inactive, but some individuals showed significant responses (Table I).

**Table I. Effect of dietary supplements on mitogen-induced gene expression ex vivo.**

| mRNA | Dietary supplements* | ΔΔCt (mean±standard deviation, n=3)** | | | | | | |
|---|---|---|---|---|---|---|---|---|
| | | Subject 1 | Subject 2 | Subject 3 | Subject 4 | Subject 5 | Subject 6 | Subject 7 |
| IL-2 | VA | **-0.98±0.04** | **-1.61±0.32** | 0.49±0.33 | 0.29±0.21 | **-1.16±0.02** | -0.26±0.20 | **-0.66±0.35** |
| | VC | -0.18±0.54 | 0.00±0.60 | *1.04±0.21* | *1.47±0.19* | **-0.80±0.13** | *1.02±0.05* | 0.07±0.27 |
| | VD | -0.13±0.07 | 0.06±0.40 | 0.00±0.15 | -0.31±0.30 | -0.45±0.61 | -0.55±0.14 | **-0.86±0.12** |
| | EGCG | -0.1±0.02 | -0.67±0.48 | *1.32±0.54* | *0.81±0.11* | **-0.99±0.33** | *1.1±0.14* | -0.41±0.29 |
| | Gen. | -0.41±0.53 | *0.90±0.44* | 0.11±9.23 | 0.08±0.04 | -0.02±1.03 | 0.00±0.27 | -0.28±0.20 |
| | Cur. | *1.47±0.59* | *2.51±0.23* | 0.31±0.14 | 0.33±0.27 | 0.03±0.20 | 0.01±0.14 | -0.01±0.21 |
| | | | | | | | | |
| IL-4 | VA | **-2.14±0.08** | **-1.56±0.34** | -0.15±0.40 | **-0.98±0.41** | -1.24±0.13 | -0.65±0.09 | **-1.15±0.39** |
| | VC | **-2.20±0.72** | **-0.86±0.62** | **-0.77±0.40** | -0.43±0.17 | -1.22±017 | **-0.77±0.09** | **-1.30±0.22** |
| | VD | -017±1.0 | 0.06±0.15 | **-0.77±0.40** | -0.43±0.17 | -1.22±0.17 | **-0.77±0.09** | **-1.30+0.22** |
| | EGCG | **-1.83±0.20** | **-1.56±0.46** | **-0.82±0.34** | **0.92±0.25** | -1.37±0.3 | **-0.9±0.01** | **-1.47±0.41** |
| | Gen. | **-1.83±0.20** | **-1.56±0.46** | **-0.82±0.34** | **-1.04±0.16** | -0.45±0.34 | -0.34±0.02 | 0.28±0.35 |
| | Cur. | *1.27±0.78* | *3.09±0.15* | -0.01±0.07 | 0.10±0.27 | 0.00±0.20 | 0.10±0.13 | 0.52±0.26 |
| | | | | | | | | |
| TNFα | VA | **-1.56±0.11** | -0.57±0.94 | 0.51±0.03 | -0.08±0.29 | **-0.76±0.34** | -0.50±0.10 | **-1.11±0.24** |
| | VC | **-1.82±0.65** | 0.10±1.14 | -0.04±0.27 | 0.09±0.16 | **-1.21±0.14** | -0.48±0.04 | -0.40±0.15 |
| | VD | -0.25±1.47 | 0.14±0.40 | -0.04±0.27 | 0.09±0.16 | **-1.21±0.14** | -0.48±0.04 | -0.40±0.15 |
| | EGCG | **-1.14±0.32** | -0.63±0.66 | 0.33±0.62 | -0.16±0.12 | **-1.22±0.29** | **-0.66±0.14** | **-0.94±0.46** |
| | Gen. | **-1.14±0.32** | **-1.01±0.04** | 0.33±0.62 | -0.16±0.12 | -0.32±0.54 | -0.02±0.20 | -0.17±0.18 |
| | Cur. | *3.52*±*1.84* | *3.64±2.26* | 0.83±0.60 | 0.4±0.15 | 0.13±0.17 | 0.07±0.01 | 0.12±0.21 |

| | | | | | | | | |
|---|---|---|---|---|---|---|---|---|
| *VA: vitamin A (final concentration=100 nM), VC: vitamin C (10 µg/mL), VD: vitamin D (100 nM), EGCG: epigallocatechin gallate (green tea) (10 µM), Gen: genistein (soybean) (2 µM), Cur.: curcumin, (turmeric) (200 nM). ** Bold: significant enhancement, underline italic: significant inhibition | | | | | | | | |

**TABLE 2: GENE PRIMER SEQUENCES**

| mRNA | TAQMAN Probe | Forward Primer | Reverse Primer |
|---|---|---|---|
| IL-2 | CTGATGAGAC AGCAACCATT GTAGAATTTC TGAA | GAACTAAAGG GATCTGAAAC AACATTC | TGTTGAGATG ATGCTTTGAC AAAA |
| | (SEQ ID No. 1) | (SEQ ID No. 2) | (SEQ ID No. 3) |
| IL-4 | CGATTCCTGAA ACGGCTCGACAGG | CACAGGCACA AGCAGCTGAT | CCTTCACAGG ACAGGAATTC AAG |
| | (SEQ ID No. 4) | (SEQ ID No. 5) | (SEQ ID No. 6) |
| TNF-α | ACTTTGCCGA GTCTGGGCAGG | TCAATCGGCCCGACTATCTC | CAGGGCAATG ATCCCAAAGT |
| | (SEQ ID No. 7) | (SEQ ID No. 8) | (SEQ ID No. 9) |
| CD32A | CTGAACCCCA GGGCACCTAC TGACG | GCTGACGGCG GCTACATG | GAGGAAGAGT CAGGTAGATG TTTTTATCA |
| | (SEQ ID No. 10) | (SEQ ID No. 11) | (SEQ ID No. 12) |
| p21 | CCACTCCAAA CGCCGGCTGA TC | TTCTGCTGTCTCTCCTCAGATTTCT | GGATTAGGGC TTCCTCTTGG A |
| | (SEQ ID No. 13) | (SEQ ID No. 14) | (SEQ ID No. 15) |
| PUMA | CCCCGCCCCA TCAATCCCA | GGGCCCAGACTGTGAATCCT | ACGTGCTCTC TCTAAACCTA TGCA |
| | (SEQ ID No. 16) | (SEQ ID No. 17) | (SEQ ID No. 18) |
| FasL | FAM-CTGAGCCATC GGTGAAACTA ACAGATAAGC A- TAMRA | TGGCAGCATC TTCACTTCTA AATG | GAAATGAGTC CCCAAAACAT CTCT |
| | (SEQ ID No. 19) | (SEQ ID No. 20) | (SEQ ID No. 21) |
| TNFSF3 | | AGGGTGTACGTCAACATCAGTCA | CACGGCCCCAAAGAAGGT |
| | | (SEQ ID No. 22) | (SEQ ID No. 23) |
| TNFSF15 | | TGCGAAGTAGGTAGCAACTGGTT | CCATTAGCTTGTCCCCTTCTTG |
| | | (SEQ ID No. 24) | (SEQ ID No. 25) |
| RNA34 | CCAAGGCCCA GCCCTCACAC A | AGCCCCCTCACTCCCAAA | GGGTGCTGTGCTTCTGTGAAC |
| | (SEQ ID No. 26) | (SEQ ID No. 27) | (SEQ ID No. 28) |

### Embodiment 2

In this embodiment of the method of the present invention, the expression of CD32A mRNA was assessed in the leukocytes of an individual. This mRNA encodes the IgG Fc receptor, and is related to antibody-dependent cell-mediated cytotoxicity (ADCC). Dietary components that increase CD32A mRNA levels would be expected to boost an individual's ADCC activity and thus provide direct anticancer activity. Alternatively, such dietary components could enhance the efficacy of recently developed expensive monoclonal antibody-based treatments (such as trastuzumab (Herceptin) or rituximab (Rituxan)) by simultaneously increasing IgG Fc receptor mRNA levels in circulating leukocytes. The method described above was employed in measuring the CD32A mRNA levels, with the exception that no stimulating agent such as phytohemagglutinin was employed. The primer sequences are shown in Table 2 above.

The dietary supplements employed were: vitamin A ("VA"; 100 nmol/L, final concentration;), vitamin C ("VC", 10 mg/mL), vitamin D ("VD", 100 nmol/L), vitamin E ("VE"; 1 IU/mL), epigallocatechin gallate (a cathecin polyphenol obtained from green tea) ("EGC" or "cathecin"; 10 mmol/L), γ-linoleic acids (polyunsaturated fatty acid in vegetable oils) ("rLA"; 1 mg/mL), genistein (soy) ("Gen"; 2 mmol/L), curcumin ("Cur"; spice turmeric) (200 nmol/L), quercetin ("Que"; plant pigments flavonoids) (100 nmol/L), aged garlic (Kyolic ("Kyo"), full strength), Agaricus ("Aga"; Kyowa, full strength), Propolis ("Pro"; 1:10 dilution), Meshimakobu ("Mesh"), Noni extract ("Noni"), and shark liver oil ("Alk"; alkoxy glycerol) (unidentified doses). All of these dietary components have reported effects on the immune system or cancer or both. In addition to the dietary components discussed in Embodiment 1 above, Vitamin E is known to have a strong effect on immune phagocytosis, and has been shown to be beneficial to animals, especially under stress, in decreasing susceptibility to infections. A lack of γ-linoleic acids results in immune system deficiency; it is an intermediate in the production of immune-supporting prostaglandins. Quercetin has been shown to boost natural killer cell activity in rats and to inhibit degranulation of mast cells, basophils and neutrophils. Aged garlic extracts have been used for decades as immune system boosters. Extracts of Agaricus mushrooms such as Agaricus blazei have been shown to have antitumor effects. Propolis, an antibiotic obtained from bee hives, contains caffeic acid phenethyl ester, which has been shown to prevent cancer formation in animal models. Propolis inhibits cancer cell growth by increasing the process of apoptosis. Meshimakobu (Phellinus linteus) is an important medicinal mushroom that contains anti-neoplastic beta-glucans; aqueous extracts thereof have been shown to suppress the proliferation of tumors. Noni extract is obtained from the fruit of the Indian mulberry (Morinda citrifolia), and contains a polysaccharide-rich substance called noni-ppt that has been shown to significantly enhance the duration of survival of mice with lung tumors. Alkoxyglycerols such as those found in shark liver oil have been employed as anti-cancer treatments and immune-enhancing agents. An additional dietary component that could be employed is fucoidan, which is obtained from seaweed and has been shown to inhibit tumor cell invasion and to boost levels of immune system components. In this embodiment, phosphate-buffered saline was employed as a control.

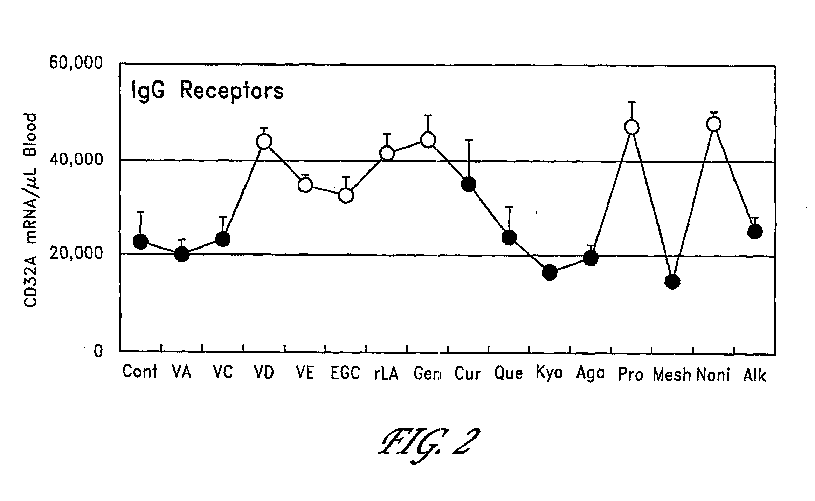

Incubation was at 37°C for 3 hours. Then CD32A mRNA was quantified, and the results are shown in Figure 2. Open circles indicate p<0.05. Each symbol is the mean ± S.D. from triplicate aliquots of 50 mL heparinized whole blood. Plasma concentrations of genistein have been reported to be 4 mmol/L at 8 hours after a single soy meal. Thus, the 3-hour incubation with 2 mmol/L genistein in whole blood is reasonably achievable.

The results in this individual indicate that the dietary components vitamins D & E, epigallocatechin gallate, γ-linoleic acid, genistein, propolis, and noni extract would be suitable for use in this individual in a diet optimized for anti-cancer activity.

### Embodiment 3

In this embodiment, heparinized whole blood of four individuals was pre-incubated with various dietary supplements at 37 °C for 1-2 hours (at the same blood concentrations as in Embodiment 2), then stimulated with 1 Gy radiation. The blood was then incubated at 37°C for 2 hours. The level of p21 mRNA was then assessed using the method described in Embodiment 1. The primer sequences are given in Table 2. p21 mRNA was selected as an apoptosis marker that would indicate the effect of each dietary component on the DNA damage response. As an alternative to p21, PUMA (p53 upregulated modulator of apoptosis) mRNA could be quantitated.

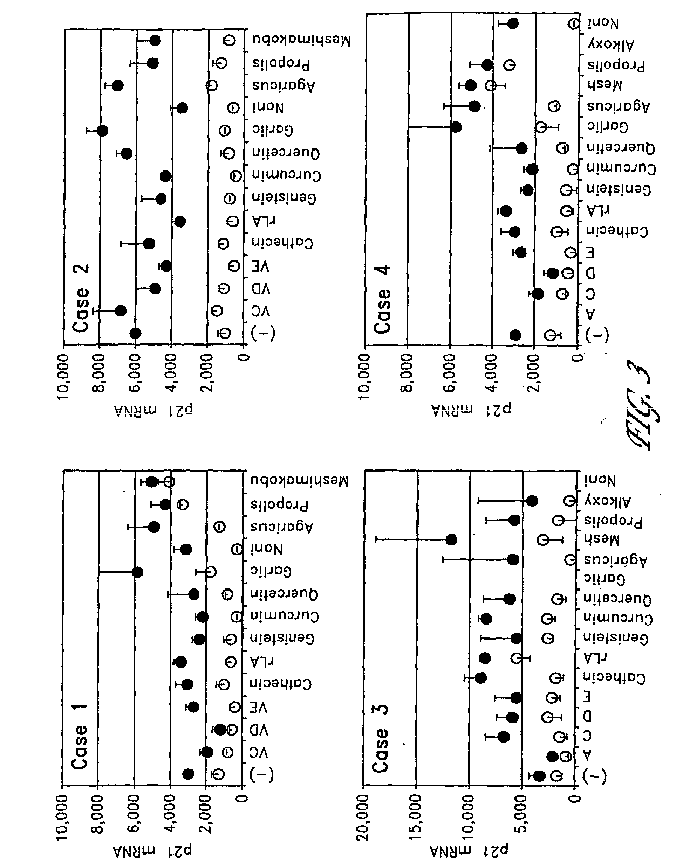

The results are shown in Figure 3. In the Figure, open circles indicate values obtained without radiation stimulus, while closed circles indicate that radiation was used. Each symbol is the mean ± S.D. from triplicate aliquots of 50 mL heparinized whole blood.

As shown in the figure, the individual responses to the dietary components varied widely. Such results could be used to design individual diets optimized for treating or preventing cancer by promoting apoptosis in neoplastic cells.

### Embodiment 4

In this embodiment, heparinized whole blood of two individuals was pre-incubated with various dietary supplements at 37 °C for 1-2 hours (at the same blood concentrations as in Embodiment 2), then stimulated phytohemagglutinin. The blood was then incubated at 37°C for 2 hours. The level of Fas ligand (FasL) mRNA was then assessed using the method described in Embodiment 1. The primer sequences are given in Table 2. Fas ligand MRNA was selected as an apoptosis marker that would indicate the effect of each dietary component on the promotion of apoptosis.

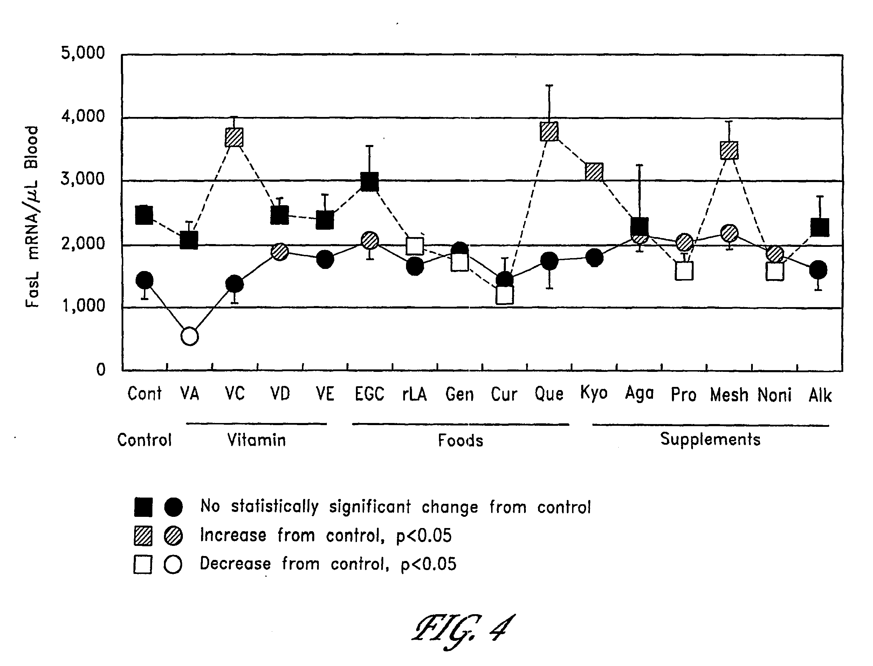

The results are shown in Figure 4. In the figure, solid symbols indicate values that do not differ significantly from control values, cross-hatched symbols indicate values that represent a significant increase over control values (p<0.05), and open symbols indicate values that represent a significant decrease compared to the control values (p<0.05). Each symbol is the mean ± S.D. from triplicate aliquots of 50 mL heparinized whole blood.

As shown in Figure 4, the individual responses to the dietary components varied considerably. Such results could be used to design individual diets optimized for treating or preventing cancer by promoting apoptosis in neoplastic cells.

### Embodiment 5

In this embodiment, heparinized whole blood of two individuals was pre-incubated with various dietary supplements at 37 °C for 30 minutes (at the same blood concentrations as in Embodiment 2), then stimulated with 1.2 µL of heat-aggregated IgG. Heat aggregated IgG (HAG) was prepared by heating 20 mg/mL human IgG (Sigma, St. Louis) in PBS at 63°C for 15 min (see Ostreiko et al., Immunol Lett. 15, 311 (1987)). The blood was then incubated at 37°C for 2 hours. The level of TNFSF3 and TNFSF15 mRNA was then assessed using the method described in Embodiment 1, with the exception that SYBR Green PCR was used to quantitate the mRNA. TNFSF3 is also known as lymphotoxin-alpha (LT alpha), tumor necrosis factor-beta (TNF-beta), and lymphotoxin-beta (LT beta). Secreted LT alpha assembles as a soluble homotrimer, LT alpha 3. Secreted LT alpha also complexes with the membrane-associated LT beta to generate two types of heterotrimers, LTalpha 1/beta and LT alpha 2/beta 1. TNFSF3 is expressed by activated naive CD4 cells, unpolarized IL-2-secreting effectors, and Thl effectors, and TNFSF3 receptors are expressed by some tumor cells. TNFSF15 is also known as TL1A and is a type II transmembrane protein belonging to the TNF superfamily. TNFSF15 is predominantly expressed in endothelial cells and its expression is inducible by TNF-α and IL-1α. TNFSF15 binds with high affinity to death receptor 3 (DR3), which is now designated TNF receptor superfamily member 25 (TNFRSF25). Depending on the cell context, ligation of DR3 by TNFSF15 can trigger one of two signaling pathways, activation of the transcription factor NF-kB or activation of caspases and apoptosis.

The primer sequences are given in Table 2 above. These mRNAs were selected as apoptosis markers that would indicate the effect of each dietary component on immune system activity and the apoptotic response.

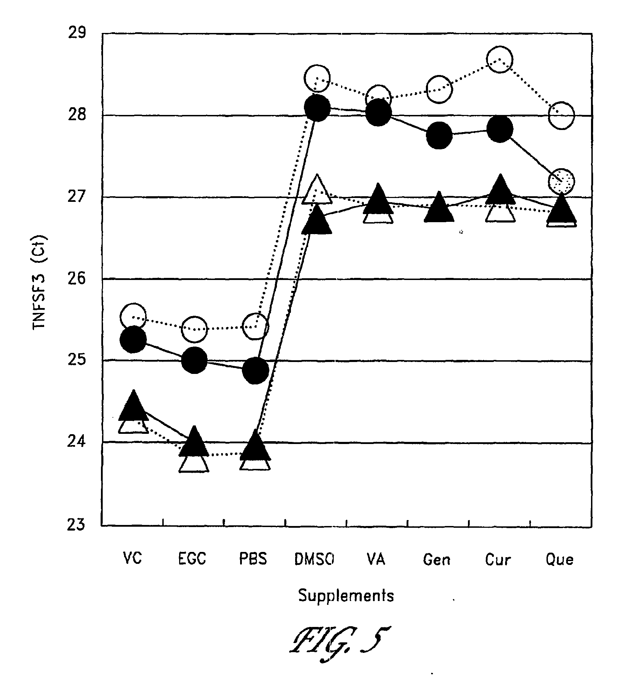

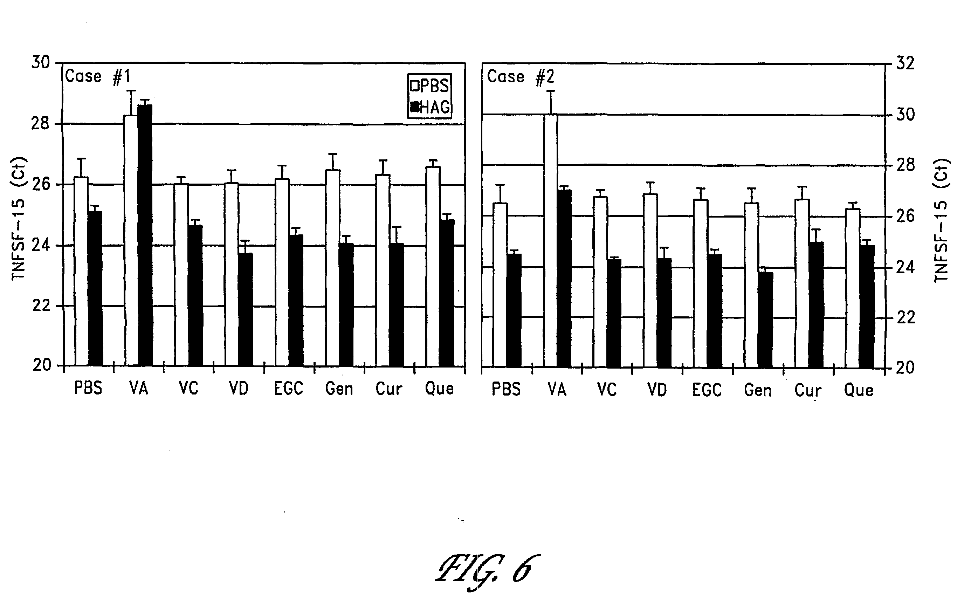

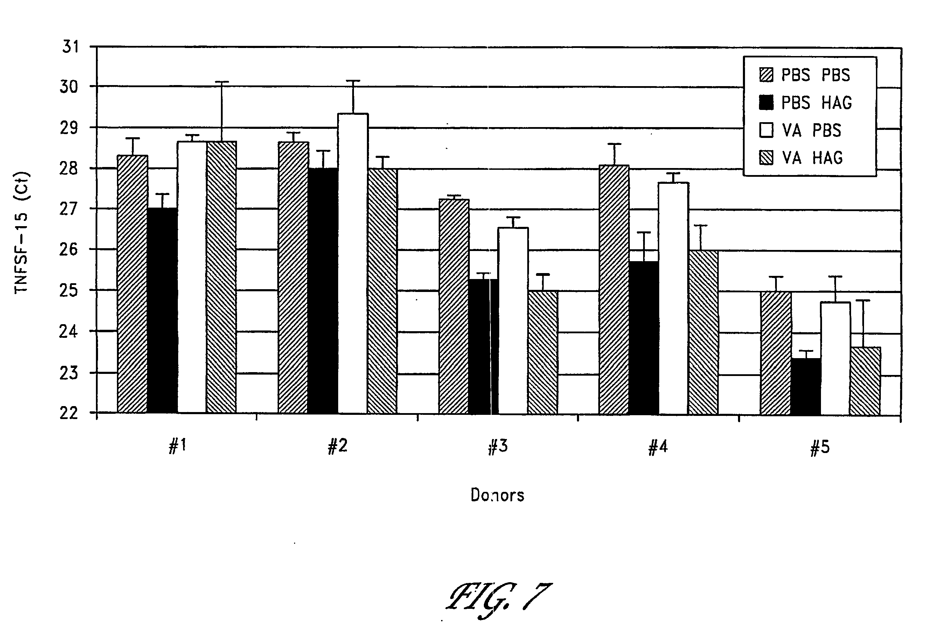

The results are shown in Figures 5, 6, and 7. In Figure 5, the open circles indicate TNFSF3 values obtained from case 1 using a PBS control, while closed circles indicate TNFSF3 values obtained in case when using a HAG stimulus. Furthermore, the gray-shaded circle indicates that HAG induced TNFSF3 expression in blood exposed to quercetin. Because TNFSF3 receptors are expressed on some cancer cells, an increase in the expression of this gene may increase apoptosis of these cells, so that quercetin would be a good candidate for inclusion in a diet having anti-cancer properties for this individual. Open and closed triangles indicate the same values from case 2. The Y-axis values in Figure 5 show the cycle threshold (Ct). Each symbol is the mean ± S.D. from triplicate aliquots of 50 mL heparinized whole blood. Figures 6 and 7 show the results of a quantitation of TNFSF15 in two cases (Figure 6) and in five further cases, with the administration of vitamin A only (Figure 7). As can be seen from Figure 6, vitamin A decreased the baseline expression of TNFSF-15, and eliminated the effect on HAG in one case (case 1). In the follow-up data shown in Figure 7, vitamin A exhibited an inhibitory action on HAG-induced TNFSF15 expression in case 1, and in two of seven cases overall (case 1 in both figures). In cases such as these cases 1, vitamin A may be useful in treating or preventing conditions in which the immune system is inappropriately activated, such as autoimmune diseases.

In the embodiments described above, dietary supplements were employed for which blood levels have been determined. However, in other embodiments of the method, various crude extracts of natural products may be screened to identify active components. By selecting appropriate mRNA targets, various functions can be addressed. The choice of the appropriate mRNA to quantitate to assess the potential effectiveness of dietary components with respect to particular conditions is well within the competence of one of skill in the art. The results demonstrate that physiological *ex vivo* gene expression analysis is a suitable methodology for the identification of the efficacy of dietary supplements in individuals. Negative responses even at high concentrations of dietary supplements may suggest that such persons are unlikely to expect a desirable augmentation or inhibition of leukocyte functions by the tested dietary supplement at the time of blood draw. Although this study employed specific dietary supplements, the method is equally applicable to other dietary components, such as specific foods.

## Claims

1. A method of assessing the potential effectiveness of a dietary component in an individual mammal against cancer or an autoimmune disorder, comprising:
exposing whole blood of the mammal to the dietary component and a stimulating agent selected from phytohemagglutinin and heat-aggregated IgG *in vitro*;
measuring the amount of an mRNA in leukocytes of the exposed whole blood, and unexposed whole blood, said mRNA being associated with the cancer or an autoimmune disorder; and
identifying the potential effectiveness of the dietary component in the mammal based on the results of the measurement, wherein a change in the amount of the mRNA determined by comparing the amount of the mRNA measured in leukocytes of the unexposed whole blood with the amount of the mRNA measured in leukocytes of the exposed whole blood correlates with the potential effectiveness of the dietary component,
wherein the mRNA is selected from mRNAs encoding interleukin-2, interleukin-4, tumor necrosis factor alpha, IgG Fc receptor, p21, Fas ligand, tumor necrosis factor superfamily member 3, and tumor necrosis factor superfamily member 15.

2. The method of claim 1, wherein the unexposed whole blood is exposed to a control vehicle before the amount of mRNA is measured.

3. The method of claim 2, wherein the control vehicle is phosphate-buffered saline or dimethyl sulfoxide.

4. The method of claim 1, wherein exposing whole blood includes addition of heparin.

5. The method of claim 1, wherein the whole blood is stimulated for 5 hours or less.

6. The method of claim 1, wherein the whole blood is stimulated for 30 minutes to 4 hours.

7. The method of claim 1, wherein the dietary component is selected from vitamin A, vitamin C, vitamin D, vitamin E, epigallocatechin gallate, gamma-linoleic acids, genistein, curcumin, quercetin, aged garlic, agaricus, propolis, meshimakobu, noni extract, alkoxyglycerol, and fucoidan.

8. The method of claim 7, wherein the potential effectiveness is potential anti-cancer effectiveness, and the mRNA is mRNA encoding an IgG Fc receptor.

9. The method of claim 7, wherein the potential effectiveness is potential potential anti-cancer or anti-autoimmune disorder effectiveness, and the mRNA is mRNA encoding a protein selected from interleukin-2, interleukin-4, tumor necrosis factor alpha, and Fas ligand, and the stimulating agent is phytohemagglutinin.

10. The method of claim 7, wherein the potential effectiveness is potential potential anti-cancer or anti-autoimmune disorder effectiveness, and the mRNA is mRNA encoding a gene product selected from tumor necrosis factor superfamily 3 and tumor necrosis factor superfamily 15, and the stimulating agent is heat-aggregated IgG.

## Patentansprüche

1. Verfahren zur Beurteilung der potenziellen Wirksamkeit eines Ernährungsbestandteils gegen Krebs oder eine Autoimmunstörung bei einem individuellen Säugetier, umfassend:
Behandeln des Ganzbluts des Säugetiers unter In-vitro-Bedingungen mit dem Ernährungsbestandteil und einem stimulierenden Mittel, das aus Phytohämagglutinin und hitzeaggregiertem IgG ausgewählt ist,
Messen der mRNA-Menge in Leukozyten des behandelten Ganzbluts und nicht behandelten Ganzbluts, wobei eine Verbindung zwischen der mRNA und dem Krebs oder einer Autoimmunstörung besteht, und
Ermitteln der potenziellen Wirksamkeit des Ernährungsbestandteils bei dem Säugetier auf Grundlage der Ergebnisse der Messung, wobei eine durch einen Vergleich der in Leukozyten des nicht behandelten Ganzbluts gemessenen mRNA mit der in Leukozyten des behandelten Ganzbluts gemessenen mRNA bestimmte Veränderung der mRNA-Menge eine Korrelation zu der potenziellen Wirksamkeit des Ernährungsbestandteils aufweist,
wobei die mRNA aus mRNAs mit dem Kode für Interleukin-2, Interleukin-4, Tumornekrosefaktor alpha, IgG Fc-Rezeptor, p21, Fas-Ligand, Tumornekrosefaktor Superfamily Member 3 und Tumornekrosefaktor Superfamily Member 15 ausgewählt ist.

2. Verfahren nach Anspruch 1, wobei das nicht behandelte Ganzblut vor der Messung der mRNA-Menge mit einem Kontrollmittel behandelt wird.

3. Verfahren nach Anspruch 2, wobei es sich bei dem Kontrollmittel um Phosphat-gepufferte Kochsalzlösung oder Dimethylsulfoxid handelt.

4. Verfahren nach Anspruch 1, wobei das Behandeln von Ganzblut das Zusetzen von Heparin einschließt.

5. Verfahren nach Anspruch 1, wobei das Ganzblut für eine Dauer von 5 Stunden oder weniger stimuliert wird.

6. Verfahren nach Anspruch 1, wobei das Ganzblut für eine Dauer von 30 Minuten bis 4 Stunden stimuliert wird.

7. Verfahren nach Anspruch 1, wobei der Ernährungsbestandteil aus Vitamin A, Vitamin C, Vitamin D, Vitamin E, Epigallokatechingallat, Gamma-Linolsäuren, Genistein, Curcumin, Quercetin, gealtertem Knoblauch, Champignon (Agaricus), Propolis (Bienenharz), Meshimakobu-Pilz, Noni-Extrakt, Alkoxylglycerin und Fucoidan ausgewählt ist.

8. Verfahren nach Anspruch 7, wobei es sich bei der potenziellen Wirksamkeit um potenzielle Wirksamkeit gegen Krebs handelt und es sich bei der mRNA um mRNA mit dem Kode für einen Ig G Fc-Rezeptor handelt.

9. Verfahren nach Anspruch 7, wobei es sich bei der potenziellen Wirksamkeit um potenzielle Wirksamkeit gegen Krebs oder Autoimmunstörungen handelt und es sich bei der mRNA um mRNA mit dem Kode für ein Protein handelt, das aus Interleukin-2, Interleukin-4, Tumornekrosefaktor alpha und Fas-Ligand ausgewählt ist, und es sich bei dem stimulierenden Mittel um Phytohämagglutinin handelt.

10. Verfahren nach Anspruch 7, wobei es sich bei der potenziellen Wirksamkeit um potenzielle Wirksamkeit gegen Krebs oder Autoimmunstörungen handelt und es sich bei der mRNA um mRNA mit dem Kode für ein Genprodukt handelt, das aus Tumornekrosefaktor Superfamily Member 3 und Tumornekrosefaktor Superfamily Member 15 ausgewählt ist, und es sich bei dem stimulierenden Miel um hitzeaggregiertes IgG handelt.

## Revendications

1. Procédé de détermination de l'efficacité potentielle d'un composant alimentaire contre le cancer ou une maladie auto-immune chez un mammifère comprenant les étapes consistant à :
- exposer le sang total du mammifère au composant alimentaire et à un agent de stimulation choisi parmi la phytohémagglutinine et les IgG agrégées par la chaleur, *in vitro*,
- mesurer la quantité d'un ARNm dans des leucocytes du sang total exposé et du sang total non exposé, cet ARNm étant associé au cancer ou à une maladie auto-immune, et
- identifier l'efficacité potentielle du composant alimentaire chez le mammifère à partir des résultats de la mesure, une variation de la quantité d'ARNm déterminée en comparant la quantité d'ARNm mesurée dans des leucocytes du sang total non exposé avec la quantité d'ARNm mesurée dans des leucocytes du sang total exposé étant en rapport avec l'efficacité potentielle du composant alimentaire,
étant précisé que l'ARNm est choisi parmi les ARNm codants pour l'interleukine-2, l'interleukine-4, le facteur de nécrose tumorale alpha, un récepteur d'IgG Fc, p21, le ligand de Fas, le membre 3 de la superfamille du facteur de nécrose tumorale et le membre 15 de la superfamille du facteur de nécrose tumorale.

2. Procédé conforme à la revendication 1,
selon lequel
le sang total non exposé est exposé à un véhicule de contrôle avant la mesure de la quantité d'ARNm.

3. Procédé conforme à la revendication 2,
selon lequel
le véhicule de contrôle est un tampon phosphate salin ou du diméthyl sulfoxide.

4. Procédé conforme à la revendication 1,
selon lequel
l'étape d'exposition du sang total renferme l'addition d'héparine.

5. Procédé conforme à la revendication 1,
selon lequel
le sang total est stimulé pendant 5 heures ou moins.

6. Procédé conforme à la revendication 1,
selon lequel
le sang total est stimulé pendant 30 minutes à 4 heures.

7. Procédé conforme à la revendication 1,
selon lequel
le composant alimentaire est choisi parmi la vitamine A, la vitamine C, la vitamine D, la vitamine E, l'épigallocatechine gallate, les acides gamma-linoléiques, la génistéine, le curcumin, la quercetine, l'ail vieilli, les agaricus, la propolis, le meshimakobu, l'extrait de noni, l'alcoxyglycérol, et le fucoidane.

8. Procédé conforme à la revendication 7,
selon lequel
l'efficacité potentielle est une efficacité potentielle anticancer et l'ARNm est un ARNm codant pour un récepteur d'IgG Fc.

9. Procédé conforme à la revendication 7,
selon lequel
l'efficacité potentielle est une efficacité potentielle anticancer ou une efficacité potentielle anti-maladie auto-immune, et l'ARNm est un ARNm codant pour une protéine choisie parmi l'interleukine-2, l'interleukine-4, le facteur de nécrose tumorale alpha, et le ligand de Fas, et l'agent de stimulation et la phytohémagglutinine.

10. Procédé conforme à la revendication 7,
selon lequel
l'efficacité potentielle est une efficacité potentielle anticancer ou une efficacité potentielle anti-maladie auto-immune, et l'ARNm est un ARNm codant pour un produit de gène choisi parmi le membre 3 de la superfamille du facteur de nécrose tumorale, et le membre 15 de la superfamille du facteur de nécrose tumorale, et l'agent de stimulation est une IgG agrégée par la chaleur.
